# EUROPEAN PATENT SPECIFICATION

(11) **EP 3 581 097 B1**
(45) Date of publication and mention of the grant of the patent: **23.09.2020**
(21) Application number: 19179036.9
(22) Date of filing: 07.06.2019
(51) Int. Cl.: A61B 5/00, A61B 17/00, A61B 5/11, A61B 5/145, A61B 90/00

(54) **A MECHANICAL SYSTEM FOR ATTACHING A MEASURING PROBE TO PROVIDE MONITORING OF TRANSPLANTED ORGANS**
MECHANISCHES SYSTEM FÜR BEFESTIGUNG DES MESSFÜHLERS ZUR ÜBERWACHUNG DER VERPFLANZTEN ORGANE
UN SYSTÈME MÉCANIQUE DE FIXATION D'UNE SONDE POUR SURVEILLER DES ORGANES GREFFÉS

(30) Priority: 14.06.2018 CZ 20180293
(43) Date of publication of application: 18.12.2019
(73) Proprietor: Ceské vysoké ucení technické v Praze, 16000 Praha 6- Dejvice (CZ)
(72) Inventor: BORTEL, Radoslav, 96001 Zvolen (SK); HOSPODKA, Jiri, 27341 Brandysek (CZ); SEBEK, Jan, 16000 Praha 6 (CZ); JANOUSEK, Libor, 14800 Praha 4 - Kunratice (CZ); MALY, Stepan, 10900 Praha 10 - Petrovice (CZ)
(74) Representative: Kratochvil, Vaclav

(56) References cited:
- WO-A1-2004/045406
- US-A- 5 487 756
- US-A1- 2004 254 432
- US-A1- 2008 287 788
- US-A1- 2013 096 412

## Description

### Technical Field

The presented solution deals with a system for attaching a measuring probe allowing to monitor the function of transplanted organs.

### Background Art

After an organ is transplanted, there is increased risk of its failure, for example because the blood inflow or outflow may become blocked. For early diagnostics, it is vital to continuously monitor an organ's condition, which may be done for instance by means of sensory implants placed directly onto an organ itself, see for example Kyriacou, P. A., M. Hickey, and J. P. Phillips. "Pulse oximetry of body cavities and organs." Engineering in Medicine and Biology Society (EMBC), 2013 35th Annual International Conference of the IEEE. IEEE, 2013. Such implants provide useful information for several hours or days after a transplantation. After this period, the risk that the transplanted organ would fail decreases and it is desirable to remove the measuring probe from the patient's body. This removal should be done without surgery, ideally simply by pulling the measuring probe out from the patient's body.

The requirement for easy removal, however, is in contrary to the requirements for a quality measurement of the markers indicating the function of a transplanted organ. Many implants use the principles of pulse oximetry or they measure the blood flow rate based on the Doppler effect, and the quality of such measurements may be negatively affected by the measuring probe movement against the monitored organ. Such movements may be eliminated by fixing the probe by surgical sutures, but this will not allow for easy removal of the probe when needed.

Therefore, many solutions facilitating probe extraction from the patient's body have been presented. Such solutions are described, for example, in document US4926875A and related WO1989006513A1, WO1992021284A1 and related US5205292, US5160338A, US6346080B1. The common feature of all these solutions is the use of a probe body in the form of a flexible strip, which is made of a biocompatible material, and which is attached by surgical sutures. This attachment is temporary, exploiting an extractible fixing wire in various ways. By pulling such extractible wire out, the sutures are released, thus allowing easy removal of the probe from the patient's body. These solutions facilitate the probe extraction; however, they have numerous setbacks. Some solutions, such as US6346080B1, US5205292, require suturing the probe to the monitored organ, which harms the organ itself. Other solutions, such as US4926875A, US5160338A, place the sutures only on the probe body and the sutures are then extracted along with the probe. The major setback of this solution, however, is that once the sutures are released, they form a free loop, which, when being extracted alongside the probe, may get stuck in the patient's body, cause injury, or make the probe removal out of the patient's body completely impossible. Suturing also complicates the application of the measuring probe - the surgeon has to position the probe to the place of its application and then make relevant sutures. Such action may be cumbersome in case the place of application is not easy to access. In addition, this increases a surgeon's workload and prolongs the surgery.

Last but not least, although the solutions mentioned above admit the application onto an organ, they are primarily intended for monitoring the blood flow through blood vessels and they are not suitably designed to monitor the condition of organs.

Yet another principle of fixation, which can be applied on biological tissue such as nerves, was presented in US5487756A. This document describes a fixation in the shape of a sleeve, which contains a longitudal slit that can be opened and the sleeve can be pulled on the monitored tissue. The slit further contains mutually intermeshed tubes, where a fixating element can be inserted, providing a long term fixation of the sleeve. The main advantage of this solution, at least in comparison with the solutions that utilize surgical sutures, is claimed to be a very good enclosure of the monitored tissue inside the sleeve, which, however, is not an advantage for the application addressed in this document. In fact, the solution presented in US5487756A is not suitable for short term monitoring of transplanted organs, because it does not contain any mechanism for easy release of the fixation and sleeve extraction without further surgical intervention in patient's body. The next disadvantage of the solution from US5487756A is a more complicated application, where the sleeve has to be applied in an opened state and it can be fixed only after its application on the tissue. Yet another disadvantage is possibility of application only on tissue/organ of longitudal shape without any ducts on its side - the application on the entire length of organs such as kidneys is not possible because the sleeve does not provide space for renal artery, vein and ureter. Other fixation means are disclosed in documents US 2008/287788 A1 and WO 2004/045406 A1.

### Summary of invention

The disadvantages mentioned above are removed by the mechanical system for attaching a measuring probe to provide monitoring of transplanted organs according to the presented : solution, defined in claim 1. The measuring probe body is made of a biocompatible material, it is strip-shaped and it consists of a sensory part, which accommodates the measuring sensors and is to be placed onto the monitored organ, and of an outlet part that leads out of patient's body. The measuring probe body is traversed by a guiding duct that allows to hold a fixing wire.

The principle of the new solution is the following arrangement. The system for attaching a measuring probe body to the monitored organ is placed in the sensory part and it is formed by at least two grips in a form of a strip. These grips are made of a soft and elastic biocompatible material. The first end of the grips is fixed from one side to the sensory part of the measuring probe body and their second end fits into the recesses made in the sensory part of the measuring probe body at the side of the measuring probe body opposite to the place where the first ends of the grips are fixed. In the part, where the second ends of the grips fit into the recesses, through-holes are created in the grips. The guiding duct is formed lengthwise through the measuring probe body at the edge with recesses and it is terminated before the distal end of the sensory part. These through-holes and the guiding duct lead one into the other so that they can hold an extractable fixing wire.

The advantage of this arrangement is that the measuring probe is easy to apply onto the monitored organ and easy to extract from the patient's body - during application, no suturing is needed, and during extraction, it eliminates the risk that the sutures might be captured in the patient's body. Another advantage is that the described arrangement is designed for monitoring of organs as opposed to many currently available solutions, which are designed for application on blood vessels.

### Brief description of drawings

The measuring probe and the method of its attachment are described by the drawings in Fig. 1 to 5:
Fig. 1 shows the measuring probe before it is applied onto the monitored organ.
Fig. 2 shows the measuring probe applied onto the monitored organ.
Fig. 3. shows the releasing of one of the grips when the fixing wire is partially extracted.
Fig. 4 shows the releasing of all grips when the fixing wire is fully extracted.
Fig. 5 shows the detail of connection of the grips and the probe body by means of the fixing wire.

### Description of embodiments

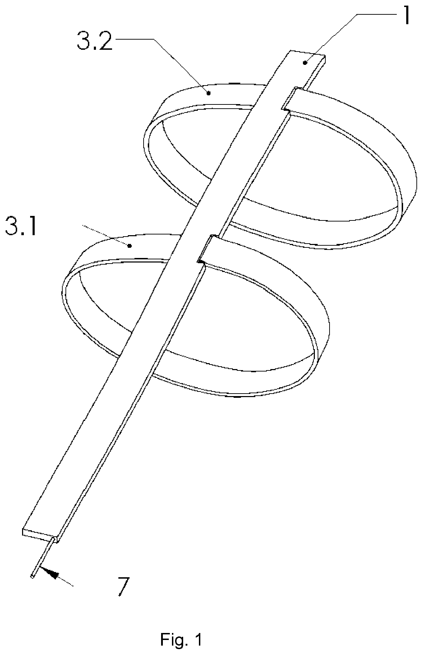

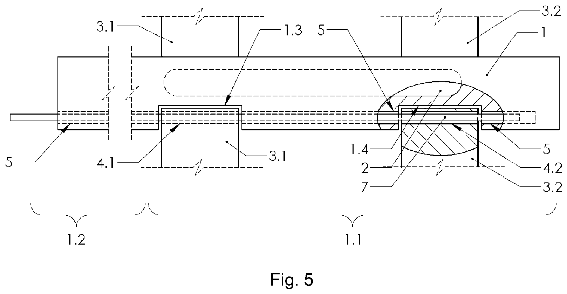

An example of the advantageous embodiment of the measuring probe is presented in Fig. 1 to 5. The measuring probe body 1 is made of a soft biocompatible material in the form of a strip and it contains a sensory part 1.1 and an output part 1.2. The sensory part 1.1 is applied onto a monitored organ 6. and it contains sensors 2 for its monitoring. The output part 1.2 leads out from a patient's body. The signals generated by the sensors 2 are led by wires or optical fibers through the output part 1.2 out from the patient's body for further evaluation. The system for the attachment of the measuring probe body 1 to the monitored organ 6 is located in the sensory part 1.1 and it is formed by at least two grips in the form of a strip. In the given example, Fig. 1 to Fig. 5 show the embodiment with two grips, formed by the first grip 3.1 and the second grip 3.2, which are made of a soft and elastic biocompatible material, such as silicone. The first end of each of the grips 3.1 and 3.2 is attached from one side to the sensory part 1.1 of the measuring probe body 1. The second end of the first grip 3.1 fits into the first recess 1.3 and the second end of the second grip 3.2 fits into the second recess 1.4, which are created in the sensory part 1.1 of the measuring probe body 1 at the side of the measuring probe body 1 opposite to the side where the ends of the grips 3.1 and 3.2 are attached. In Fig. 1 to Fig. 5, the recesses 1.3 and 1.4 are shown coaxially with the attached ends of the grips 3.1 and 3.2. but this is only an example because the recesses 1.3 and 1.4 do not necessarily need to be coaxial - this depends on the specific application of the measuring probe. In the part where the second ends of the grips 3.1 and 3.2 fit into the recesses 1.3 and 1.4 the first through-hole 4.1 is created in the first grip 3.1 and the second through-hole 4.2 is created in the second grip 3.2, which are both aligned with a guiding duct 5 created lengthwise along the measuring probe body 1 at the edge with the recesses 1.3, 1.4, and terminated in the measuring probe body 1 before the distal end of the sensory part. The first through-hole 4.1, the second through-hole 4.2 and the guiding duct 5, which lead one into the other, hold an extractible fixing wire 7. The fixing wire 7 diameter is chosen such that the wire cannot slip out by itself, but is extractable by hand. In practice, the fixing wire 7 diameter will be slightly less than the diameter of the guiding duct 5 and the through-holes 4.1 and 4.2. The length of the grips 3.1 and 3.2 is chosen based on the size of the organ 6, for which the measuring probe is intended. It must safely fix the measuring probe to the organ 6 without exerting excessive pressure on it.

By inserting the fixing wire 7 simultaneously into the guiding duct 5 of the measuring probe body 1, the first through-hole 4.1 of the first grip 3.1 and the second through-hole 4.2 of the second grip 3.2 the second ends of the grips 3.1 and 3.2 become fixed to the measuring probe body 1, and this fixation does not require surgical suturing. The fixing wire 7 can also be extracted, which results in releasing the second ends of the grips 3.1 and 3.2 from the measuring probe body 1, the measuring probe will no longer be fixed to the monitored organ 6, and it can be removed from the patient's body.

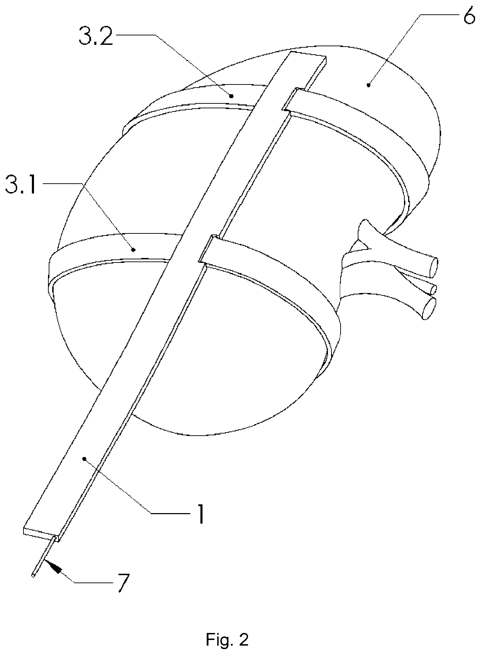

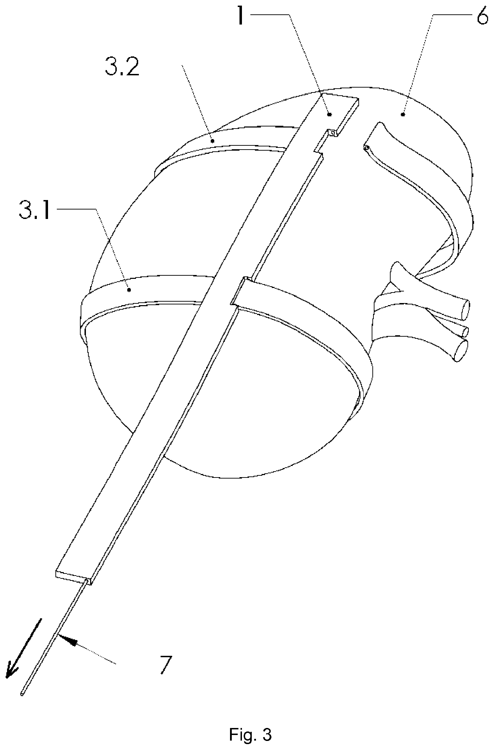

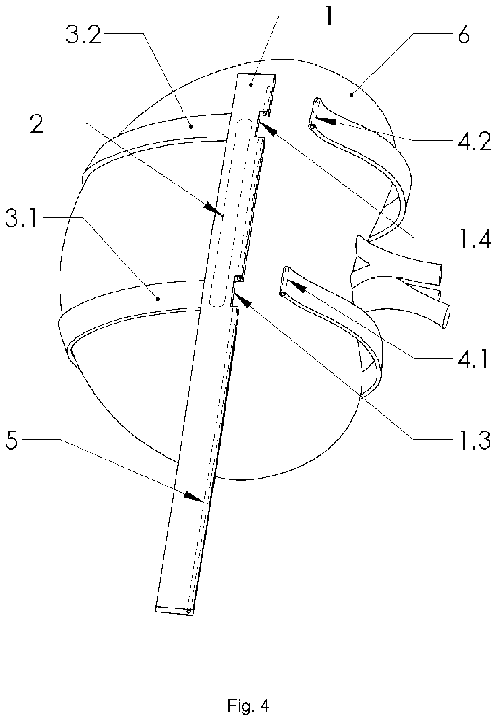

Before the application of the measuring probe onto the monitored organ 6, the second ends of the first grip 3.1 and the second grip 3.2 are fixed to the measuring probe body 1, by inserting the fixing wire 7. When the measuring probe is applied, the grips are simply pulled onto the monitored organ 6, Fig. 2, which allows for a quick, simple but also efficient fixation of the measuring probe at the place of its application. This action may be done easily and quickly and it does not require suturing. When extracting, the fixing wire 7 is first pulled out from the measuring probe body 1. This releases the grips 3.1 and 3.2, Fig. 3 and Fig. 4, and the measuring probe can be extracted from the patient's body without the risk of capturing any sutures inside the patient's body.

### Industrial applicability

The presented solution can be used for time-limited monitoring of organs after transplantation surgeries.

## Claims

1. A mechanical system for attaching a measuring probe to provide monitoring of transplanted organs, the mechanical system comprising the said measuring probe (1), where the measuring probe body (1) is made of a soft biocompatible material, it is strip-shaped and it consists of a sensory part (1.1), which contains the measuring sensors (2) and is to be placed onto a monitored organ (6), and of an output part (1.2) providing an output from a patient's body, while a guiding duct (5), where a fixing wire (7) is placed, leads through the measuring probe body (1) **characterized by the fact** that the mechanical system for attaching the measuring probe body (1) to the monitored organ (6) is placed in the sensory part (1.1) and it is formed by at least two grips in a form of a strip, specifically by a first grip (3.1), and a second grip (3.2), which are made of a soft and elastic biocompatible material, while first ends of the grips (3.1, 3.2) are fixed from one side to the sensory part (1.1) of the measuring probe body (1) and where a second end of the first grip (3.1) fits into a first recess (1.3) and a second end of the second grip (3.2) fits into a second recess (1.4), while these recesses are created in the sensory part (1.1) of the measuring probe body (1) at the side of the measuring probe body (1) opposite to the side where the first ends of the grips (3.1, 3.2) are fixed, and in the part where the second ends of the grips (3.1, 3.2) fit into the recesses (1.3, 1.4), a first through-hole (4.1) is created in the first grip (3.1), and a second through-hole (4.2) is created in the second grip (3.2), and the guiding duct (5) is created lengthwise through the measuring probe body (1) at the edge with the recesses (1.3, 1.4), and it is terminated in the measuring probe body (1) before the distal end of the sensory part (1.1), while the first through-hole (4.1), the second through-hole (4.2), and the guiding duct (5) lead one into the other so that they can hold an extractable fixing wire (7).

## Patentansprüche

1. Mechanisches System zur Befestigung einer Messsonde zur Überwachung von Organen, das mechanische System umfasst eine Messsonde (1), wo der Körper der Messsonde (1) aus einem weichen biokompatiblen Material in Form eines Streifens besteht, und einen sensorischen Teil (1.1) zur Platzierung an dem zu überwachenden Organ (6), in dem Messsensoren (2) angeordnet sind, und einen Ausführungsteil (1.2) zur Ausführung außerhalb des Patienten umfasst, wobei durch den Körper der Messsonde (1) ein Führungskanal (5) zur Einführung eines Fixierungsdrahts (7) führt, **gekennzeichnet dadurch, dass** das mechanische System zur Befestigung des Körpers der Messsonde (1) an dem zu überwachenden Organ (6) im sensorischen Teil (1.1) angeordnet ist und durch mindestens zwei Griffe in Form eines Streifens gebildet ist, und dies durch ersten Griff (3.1) und zweiten Griff (3.2), die aus einem weichen und flexiblen biokompatiblen Material bestehen, deren ein Ende auf einer Seite am sensorischen Teil (1.1) des Körpers der Messsonde (1) befestigt ist und das zweite Ende des ersten Griffes (3.1) in die erste Einkerbung (1.3) eingreift und das zweite Ende des zweiten Griffes (3.2) in die zweite Einkerbung (1.4) eingreift, die im sensorischen Teil (1.1) des Körpers der Messsonde (1) auf der gegenüberliegenden Seite des Körpers der Messsonde (1) ausgebildet sind, als die Enden der Griffe (3.1, 3.2) befestigt sind, wobei im Teil, wo die zweiten Enden in die Einkerbungen (1.3, 1.4) eingreifen, ist im ersten Griff (3.1) ein durchgehender Hohlraum (4.1) ausgebildet und im zweiten Griff (3.2) ist ein zweiter durchgehender Hohlraum (4.2) ausgebildet und der Führungskanal (5) ist entlang des Körpers der Messsonde (1) am Rand mit den Einkerbungen (1.3, 1.4) ausgebildet und im Körper der Messsonde (1) vor dem distalen Ende des sensorischen Teiles (1.1) beendet, wobei der erste durchgehende Hohlraum (4.1), der zweite durchgehende Hohlraum (4.2) und der Führungskanal (5) miteinander verbunden sind und in ihnen ist der Fixierungsdraht (7) herausnehmbar angeordnet.

## Revendications

1. Un système mécanique pour fixer une sonde de mesure pour surveiller l'état des organes, le système mécanique est composé d'une sonde (1) de mesure, où le corps de la sonde (1) de mesure est en matériau biocompatible souple en forme de bande et se compose d'une partie (1.1) capteur à placer sur l'organe (6) surveillé dans laquelle se trouvent des capteurs (2) de mesure et d'une partie (1.2) de sortie pour la sortie à l'extérieur du corps du patient, un canal (5) de guidage pour recevoir un fil (7) de fixation traversant le corps de la sonde (1) de mesure, **caractérisé en ce que** le système mécanique pour fixer le corps (1) de la sonde de mesure à l'organe (6) surveillé est placé dans la partie (1.1) capteur et est constitué d'au moins deux préhensions en forme de ruban, et ce, d'une première préhension (3.1) et d'une deuxième préhension (3.2), en matériau biocompatible souple, dont une extrémité est fixée d'un côté à la partie (1.1) capteur du corps (1) de la sonde de mesure et où l'autre extrémité de la première préhension (3.1) s'insère dans une première encoche (1.3) et l'autre extrémité de la deuxième préhension (3.2) s'insère dans une seconde encoche (1.4) qui sont formées dans la partie (1.1) capteur du corps (1) de la sonde de mesure sur le côté opposé du corps (1) de la sonde de mesure au côté où les extrémités des préhensions (3.1, 3.2) sont fixées, en même temps, dans la partie où les autres extrémités s'insèrent dans les encoches (1.3, 1.4), dans la première préhension (3.1) une première cavité (4.1) traversante est formée et dans la deuxième préhension (3.2) une deuxième cavité (4.2) traversante est formée et le canal (5) de guidage est formé le long du corps (1) de la sonde de mesure près du bord avec les encoches (1.3, 1.4) et se termine dans le corps (1) de la sonde de mesure devant l'extrémité distale de la partie (1.1) capteur, la première cavité (4.1) traversante, la seconde cavité (4.2) traversante et le canal (5) de guidage s'enchaînant et un fil (7) de fixation y est logé de manière amovible.
